# EUROPEAN PATENT APPLICATION

(11) **EP 4 790 714 A1**
(43) Date of publication of application: **12.08.2026**
(21) Application number: 25869574.1
(22) Date of filing: 05.08.2025
(51) Int. Cl.: G16C 10/00

(54) **METHOD AND APPARATUS FOR DETERMINING POSITION OF COHESIVE ZONE IN BLAST FURNACE, DEVICE, AND STORAGE MEDIUM**

(30) Priority: 19.12.2024 CN 202411877341
(71) Applicant: INSTITUTE OF RESEARCH OF IRON AND STEEL, JIANGSU PROVINCE/SHA-STEEL, CO. LTD, Suzhou, Jiangsu 215625 (CN); Jiangsu Shagang Steel Co., Ltd., Suzhou, Jiangsu 215625 (CN); Jiangsu Shagang Group Co., Ltd., Suzhou, Jiangsu 215625 (CN)
(72) Inventor: DU, Ping, Suzhou, Jiangsu 215625 (CN); ZHAO, Huatao, Suzhou, Jiangsu 215625 (CN); ZHANG, Shaobo, Suzhou, Jiangsu 215625 (CN); MIAO, Haishan, Suzhou, Jiangsu 215625 (CN)
(74) Representative: M. Zardi & Co S.A.
(86) International application number: PCT/CN2025/112781
(87) International publication number: WO 2026/129671

(57) **Abstract**

The present application relates to the technical field of blast furnace smelting, and discloses a method, apparatus, device and storage medium for determining the position of a cohesive zone in a blast furnace. The method includes: based on a charging start signal and a charging end signal of a current charging operation, performing a stockline scan to obtain stockline coordinates and a descending velocity of furnace burden; based on the stockline coordinates and blast furnace characteristics, determining a first boundary condition and constructing an initial grid structure; based on the stockline coordinates and the descending velocity of the furnace burden, updating the initial grid structure to obtain a target grid structure; causing the furnace burden in the blast furnace to undergo reactions, and when a plurality of furnace burden performance parameters corresponding to each grid node in the target grid structure converge, obtaining a distribution situation of each grid cell in the target grid structure and generating a temperature field distribution map; and based on the plurality of furnace burden performance parameters corresponding to each grid node in the target grid structure and the temperature field distribution map, determining the position of the cohesive zone in the blast furnace. The present application improves the accuracy of determining the position of the cohesive zone and reflects the dynamic changes inside the blast furnace.

## Description

### CROSS-REFERENCE TO RELATED APPLICATIONS

The present application claims the priority of the Chinese Patent Application No. 202411877341.7, filed with the China National Intellectual Property Administration on December 19th, 2024, entitled "METHOD, APPARATUS, DEVICE AND STORAGE MEDIUM FOR DETERMINING POSITION OF COHESIVE ZONE IN BLAST FURNACE", the entire contents of which are incorporated herein by reference.

### TECHNICAL FIELD

The present application relates to the technical field of blast furnace smelting, and in particular, to a method, an apparatus, a device and a storage medium for determining a position of a cohesive zone in a blast furnace.

### BACKGROUND ART

The cohesive zone of a blast furnace refers to the area in the blast furnace where, as the furnace burden descends to a certain depth, the ore begins to soften and partially melt to form primary slag due to the gradual increase in temperature. In this area, gangue in the ore undergoes physical and chemical changes such as moisture evaporation, carbonate decomposition, ore softening and melting. The area above the cohesive zone is mainly a solid layer for drying, preheating and initial chemical reactions, and a flowing molten layer, also referred to as a dripping zone, is formed below the cohesive zone, where iron oxides in the ore are further reduced and react with coke to finally produce molten iron and slag. The position of the cohesive zone in the blast furnace is crucial for blast furnace operation, as it directly affects heat energy utilization, chemical reaction efficiency, blast furnace lining service life and other aspects inside the blast furnace. Therefore, how to determine the position of the cohesive zone in the blast furnace is a problem to be solved.

At present, equipment such as an infrared thermal imager can be used to scan the blast furnace shell from the outside and infer the position of the cohesive zone according to temperature distribution. However, infrared detection mainly relies on heat radiated from the blast furnace shell, and its ability to penetrate the blast furnace shell is limited, which can only provide apparent temperature and is difficult to accurately reflect the actual temperature distribution in the deep part of the blast furnace. This results in poor accuracy in determining the position of the cohesive zone and failure to reflect the dynamic changes inside the blast furnace.

### SUMMARY

In view of the above, the present application provides a method, an apparatus, a device and a storage medium for determining a position of a cohesive zone in a blast furnace, so as to solve the problem of accurately determining the position of the cohesive zone in the blast furnace.

In a first aspect, the present application provides a method for determining a position of a cohesive zone in a blast furnace, the method including:
based on a charging start signal and a charging end signal of a current charging operation, performing a stockline scan to obtain stockline coordinates and a descending velocity of furnace burden for the current charging operation, wherein the stockline coordinates include coordinates of each point located on a top surface layer of the blast furnace after the end of the current charging operation;
based on the stockline coordinates and blast furnace characteristics, determining a first boundary condition and constructing an initial grid structure based on the first boundary condition, wherein the initial grid structure is obtained by meshing a physical model of the blast furnace under the constraint of the first boundary condition;
based on the stockline coordinates and the descending velocity of the furnace burden, updating the initial grid structure to obtain a target grid structure for the current charging operation;
causing furnace burden inside the blast furnace to undergo solid-liquid-gas three-phase flow involving mass, momentum and heat transfer, and chemical reactions, and when a plurality of furnace burden performance parameters corresponding to each grid node in the target grid structure converge, obtaining a distribution situation of each grid cell in the target grid structure and generating a temperature field distribution map, wherein the distribution situation comprises solid temperature, gas temperature, flow rate and pressure in an area where the grid cell is located; and
based on the plurality of furnace burden performance parameters corresponding to each grid node in the target grid structure and the temperature field distribution map, determining the position of the cohesive zone in the blast furnace after the current charging operation.

The method for determining the position of the cohesive zone in the blast furnace provided by the embodiment of the present application obtains the stockline coordinates and the descending velocity of the furnace burden for the current charging operation by performing real-time stockline scanning according to the charging start signal and the charging end signal, thereby updating the initial grid structure constructed based on the first boundary condition by virtue of the stockline coordinates and the descending velocity of the furnace burden to obtain the target grid structure. By simulating solid-liquid-gas three-phase flow, heat and mass transfer, and chemical reactions in the grid structure corresponding to the current charging operation, changes of the furnace burden with time and space are taken into account, which enables a deeper understanding of the complex physical and chemical changes inside the blast furnace to further generate the temperature field distribution map. The position of the cohesive zone can be determined more accurately by synthesizing the furnace burden performance parameters and the temperature field distribution map, which provides strong data support for production scheduling and process optimization, improves the accuracy in determining the position of the cohesive zone, and simultaneously reflects the dynamic changes inside the blast furnace.

In an optional embodiment, the step of performing a stockline scan based on the charging start signal and the charging end signal of the current charging operation to obtain the stockline coordinates and the descending velocity of the furnace burden for the current charging operation includes:
when a stock flow valve of the blast furnace is opened, acquiring the charging start signal of the current charging operation;
performing a stockline scan based on the charging start signal to obtain initial stockline coordinates for the current charging operation;
when a sounding rod of the blast furnace is lifted, acquiring the charging end signal of the current charging operation;
performing a stockline scan based on the charging end signal to obtain the stockline coordinates of the furnace burden for the current charging operation; and
determining the descending velocity of the furnace burden for the current charging operation based on the stockline coordinates of the furnace burden for the current charging operation, the initial stockline coordinates, and a time difference between the charging start signal and the charging end signal.

The method for determining the position of the cohesive zone in the blast furnace provided by the embodiment of the present application obtains the stockline coordinates respectively by means of scanning through real-time monitoring of the charging start signal and the charging end signal, and determines the descending velocity of the furnace burden according to the stockline coordinates obtained by two scans, which provides support for subsequent determination of the position of the cohesive zone.

In an optional embodiment, the step of updating the initial grid structure based on the stockline coordinates and the descending velocity of the furnace burden to obtain the target grid structure for the current charging operation includes:
determining grid parameters of each grid node in the initial grid structure, wherein the grid parameters comprise a radial distance, a vertical distance, a fluid radial velocity and a fluid vertical velocity;
for any grid node in the initial grid structure that corresponds to the stockline coordinates, causing the grid node to descend and determining grid parameters of a descended grid node obtained after the descent;
taking the descended grid node as a new grid node and repeating the process of descending the grid node and acquiring the grid parameters of the descended grid node until the grid node or the descended grid node exceeds a physical domain, thereby obtaining grid parameters of at least one descended grid node corresponding to the grid node; and
updating the grid parameters of each grid node in the initial grid structure based on the grid parameters of the at least one descended grid node obtained during the descending process for each grid node in the initial grid structure that corresponds to the stockline coordinates, to obtain the target grid structure.

The method for determining the position of the cohesive zone in the blast furnace provided by the embodiment of the present application determines the grid parameters of each descended grid node in the descending process by descending the grid nodes on the top stockline in the initial grid structure, thereby updating the grid parameters of each grid node in the initial grid structure according to the grid parameters of the descended grid nodes obtained during the descending process for each grid node on the top stockline. This simulates the descending process of the furnace burden with time and space, can reflect the dynamic change situation inside the blast furnace, and obtains the target grid structure conforming to the actual situation of the current charging operation, which provides support for subsequent determination of the position of the cohesive zone.

In an optional embodiment, the step of determining the grid parameters of each grid node in the initial grid structure includes:
performing coordinate transformation on the stockline coordinates to obtain grid parameters of each point in the stockline coordinates; and
determining the grid parameters of each grid node in the initial grid structure based on the grid parameters of each point in the stockline coordinates.

The method for determining the position of the cohesive zone in the blast furnace provided by the embodiment of the present application ensures that the physical properties of each point match the actual state of the furnace burden by converting the stockline coordinates into grid parameters, which helps to grasp the internal conditions of the blast furnace in more detail. In addition, the grid parameters of each grid node in the initial grid structure are determined based on the grid parameters of each point in the stockline coordinates, so as to obtain the initial grid structure with complete grid node information.

In an optional embodiment, the step of causing any grid node corresponding to the stockline coordinates in the initial grid structure to descend and determining the grid parameters of the descended grid node obtained after the descent includes:
determining a descent time of the grid node;
determining a radial distance of the descended grid node based on the radial distance of the grid node, the fluid radial velocity, and the descent time;
determining a vertical distance of the descended grid node based on the vertical distance of the grid node, the fluid vertical velocity, and the descent time;
determining a grid cell to which the descended grid node belongs in the initial grid structure based on the radial distance and the vertical distance of the descended grid node;
determining four distances between the descended grid node and four vertex grid nodes of the grid cell based on the grid parameters of the grid node, the radial distance and the vertical distance of the descended grid node, and the grid parameters of the four vertex grid nodes of the grid cell; and
determining the fluid radial velocity and the fluid vertical velocity of the descended grid node based on the grid parameters of the four vertex grid nodes of the grid cell and the four distances.

The method for determining the position of the cohesive zone in the blast furnace provided by the embodiment of the present application can simulate the sinking behavior of the furnace burden inside the blast furnace by calculating the grid parameters of the descended grid node obtained after the descent of the grid node, which provides intuitive visual and quantitative data support for understanding the dynamic changes inside the blast furnace.

In an optional embodiment, the step of causing furnace burden inside the blast furnace to undergo solid-liquid-gas three-phase flow involving mass, momentum and heat transfer, and chemical reactions, and when the plurality of furnace burden performance parameters corresponding to each grid node in the target grid structure converge, obtaining the distribution situation of each grid cell in the target grid structure and generating the temperature field distribution map includes:
determining a second boundary condition;
causing the furnace burden inside the blast furnace to undergo the solid-liquid-gas three-phase flow, heat and mass transfer, and chemical reactions for the first time to obtain the plurality of furnace burden performance parameters corresponding to each grid node in the target grid structure and the distribution situation of each grid cell after the first solid-liquid-gas three-phase flow, heat and mass transfer, and chemical reactions; and
repeating the process of causing the furnace burden inside the blast furnace to undergo the solid-liquid-gas three-phase flow, heat and mass transfer, and chemical reactions until the plurality of furnace burden performance parameters corresponding to each grid node reach convergence under the constraint of the second boundary condition, thereby obtaining the distribution situation of each grid cell in the target grid structure and generating the temperature field distribution map.

The method for determining the position of the cohesive zone in the blast furnace provided by the embodiment of the present application obtains the distribution situation of each grid cell by setting the second boundary condition and repeatedly performing the simulation of three-phase flow mass transfer and chemical reactions until the furnace burden performance parameters converge, so as to generate the temperature field distribution map. The temperature field distribution map intuitively shows the spatial distribution of temperature inside the blast furnace, which provides support for subsequent determination of the position of the cohesive zone.

In an optional embodiment, the step of determining the position of the cohesive zone in the blast furnace after the current charging operation based on the plurality of furnace burden performance parameters corresponding to each grid node in the target grid structure and the temperature field distribution map includes:
performing high-temperature droplet performance detection based on the plurality of furnace burden performance parameters corresponding to each grid node in the target grid structure to obtain an initial softening temperature and an initial dripping temperature of the furnace burden; and
based on temperatures of all grid cells in the target grid structure in the temperature field distribution map, taking all grid cells whose temperatures fall between the initial softening temperature and the initial dripping temperature as the position of the cohesive zone in the blast furnace after the current charging operation.

The method for determining the position of the cohesive zone in the blast furnace provided by the embodiment of the present application can predict the initial softening temperature and the initial dripping temperature of the furnace burden by performing high-temperature droplet performance detection under the condition that the plurality of furnace burden performance parameters corresponding to each grid node in the target grid structure are used as detection environment parameters. Thus, the grid cells in the target grid structure whose temperatures are between the two temperatures are determined as the position of the cohesive zone, achieving accurate determination of the position of the cohesive zone.

In a second aspect, the present application provides an apparatus for determining a position of a cohesive zone in a blast furnace, the apparatus including:
a scanning module configured to perform a stockline scan based on a charging start signal and a charging end signal of a current charging operation to obtain stockline coordinates and a descending velocity of furnace burden for the current charging operation, wherein the stockline coordinates comprise coordinates of each point located on a top surface layer of the blast furnace after the end of the current charging operation;
a constructing module configured to determine a first boundary condition based on the stockline coordinates and blast furnace characteristics, and to construct an initial grid structure based on the first boundary condition, wherein the initial grid structure is obtained by meshing a physical model of the blast furnace under the constraint of the first boundary condition;
an updating module configured to update the initial grid structure based on the stockline coordinates and the descending velocity of the furnace burden to obtain a target grid structure for the current charging operation;
a generating module configured to cause furnace burden inside the blast furnace to undergo solid-liquid-gas three-phase flow involving mass, momentum and heat transfer, and chemical reactions, and when a plurality of furnace burden performance parameters corresponding to each grid node in the target grid structure converge, to obtain a distribution situation of each grid cell in the target grid structure and generate a temperature field distribution map, wherein the distribution situation includes solid temperature, gas temperature, flow rate and pressure in an area where the grid cell is located; and
a determining module configured to determine the position of the cohesive zone in the blast furnace after the current charging operation based on the plurality of furnace burden performance parameters corresponding to each grid node in the target grid structure and the temperature field distribution map.

In a third aspect, the present application provides a computer device, including a memory and a processor that are communicatively connected to each other, wherein the memory stores computer instructions, and the processor executes the computer instructions to perform the method for determining a position of a cohesive zone in a blast furnace according to the first aspect or any one of the optional embodiment thereof.

In a fourth aspect, the present application provides a computer-readable storage medium, wherein the computer-readable storage medium stores computer instructions that, when executed by a computer, cause the computer to perform the method for determining a position of a cohesive zone in a blast furnace according to the first aspect or any one of the optional embodiment thereof.

In a fifth aspect, the present application provides a computer program product, comprising computer instructions that, when executed by a computer, cause the computer to perform the method for determining a position of a cohesive zone in a blast furnace according to the first aspect or any one of the optional embodiment thereof.

### BRIEF DESCRIPTION OF THE DRAWINGS

To more clearly illustrate the specific embodiments of the present application or the technical solutions in the prior art, the accompanying drawings required for describing the specific embodiments or the prior art will be briefly introduced below. Obviously, the accompanying drawings in the following description show some embodiments of the present application, and those skilled in the art can obtain other accompanying drawings based on these drawings without exerting creative efforts.
FIG. 1 is a flow chart of a method for determining a position of a cohesive zone in a blast furnace according to an embodiment of the present application;
FIG. 2 is a schematic diagram of an initial grid structure according to an embodiment of the present application;
FIG. 3 is a schematic diagram of the distribution of a stockline at the top of a blast furnace according to an embodiment of the present application;
FIG. 4 is a schematic diagram of a grid cell according to an embodiment of the present application;
FIG. 5 is a schematic diagram of a target grid structure according to an embodiment of the present application;
FIG. 6 is a schematic diagram of a cohesive zone in a blast furnace according to an embodiment of the present application;
FIG. 7 is a flow chart of another method for determining a position of a cohesive zone in a blast furnace according to an embodiment of the present application;
FIG. 8 is a structural block diagram of an apparatus for determining a position of a cohesive zone in a blast furnace according to an embodiment of the present application; and
FIG. 9 is a schematic diagram of a hardware structure of a computer device according to an embodiment of the present application.

### DETAILED DESCRIPTION OF THE EMBODIMENTS

To make the objectives, technical solutions and advantages of the embodiments of the present application clearer, the technical solutions in the embodiments of the present application will be described clearly and completely below in conjunction with the accompanying drawings in the embodiments of the present application. Obviously, the described embodiments are some, but not all, embodiments of the present application. All other embodiments obtained by those skilled in the art based on the embodiments of the present application without creative efforts shall fall within the protection scope of the present application.

The position of the cohesive zone in a blast furnace is crucial for blast furnace operation, as it directly affects heat energy utilization, chemical reaction efficiency, blast furnace lining service life and other aspects inside the blast furnace. At present, equipment such as an infrared thermal imager can be used to scan the blast furnace shell from the outside and infer the position of the cohesive zone according to temperature distribution. This method is difficult to accurately reflect the actual temperature distribution in the deep part of the blast furnace, resulting in poor accuracy in determining the position of the cohesive zone and failure to reflect the dynamic change situation inside the blast furnace. The embodiments of the present application obtain the stockline coordinates and the descending velocity of the furnace burden for the current charging operation by performing real-time stockline scanning according to the charging start signal and the charging end signal, and update the initial grid structure to obtain the target grid structure. By simulating solid-liquid-gas three-phase flow, heat and mass transfer, and chemical reactions, changes of the furnace burden with time and space are taken into account, which enables a deeper understanding of the complex physical and chemical changes inside the blast furnace to further generate the temperature field distribution map. The position of the cohesive zone can be determined more accurately by synthesizing the furnace burden performance parameters and the temperature field distribution map, which improves the accuracy in determining the position of the cohesive zone and simultaneously reflects the dynamic changes inside the blast furnace.

According to an embodiment of the present application, an embodiment of a method for determining the position of a cohesive zone in a blast furnace is provided. It should be noted that the steps shown in the flow chart of the accompanying drawings can be executed in a computer system such as a set of computer-executable instructions. Although a logical order is shown in the flow chart, in some cases, the steps shown or described can be executed in an order different from that herein.

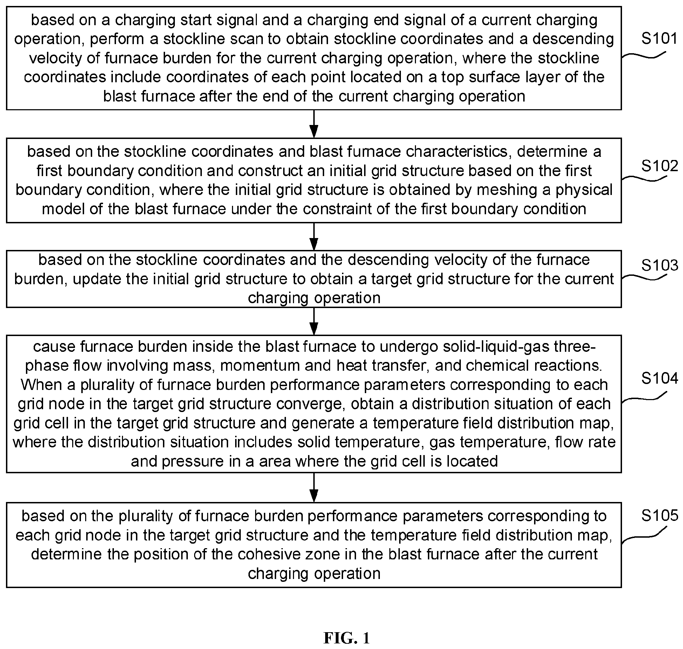

A method for determining a position of a cohesive zone in a blast furnace is provided in this embodiment, which can be applied to electronic equipment. FIG. 1 is a flow chart of the method for determining the position of a cohesive zone in a blast furnace according to an embodiment of the present application. As shown in FIG. 1, the process includes the following steps:
Step S101, based on a charging start signal and a charging end signal of a current charging operation, perform a stockline scan to obtain stockline coordinates and a descending velocity of furnace burden for the current charging operation, where the stockline coordinates include coordinates of each point located on a top surface layer of the blast furnace after the end of the current charging operation. Specifically, the furnace burden for blast furnace charging includes coke and ore. With the combustion of coke in the tuyere raceway at the bottom of the blast furnace and the discharge of molten iron and slag out of the blast furnace, the furnace burden in the blast furnace descends continuously. To keep the blast furnace in continuous operation, charging must be carried out continuously at the top of the blast furnace, and coke or ore forms the top stockline of the blast furnace in each charging operation. Optionally, an on-line laser stockline scanning device for the blast furnace can be adopted for the stockline scan, and only the top stockline of the blast furnace needs to be scanned in each scan. By performing the stockline scan on the furnace burden being charged, the coordinates of each point on the coke stockline or ore stockline can be obtained, and the descending velocity of coke or ore can be derived from the coordinates.

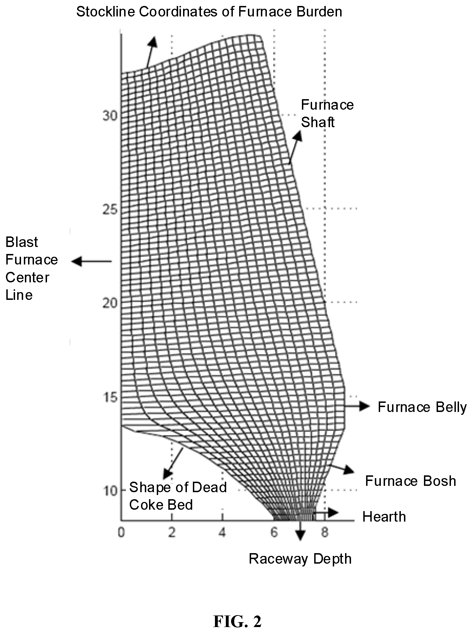

Step S102, based on the stockline coordinates and blast furnace characteristics, determine a first boundary condition and construct an initial grid structure based on the first boundary condition, where the initial grid structure is obtained by meshing a physical model of the blast furnace under the constraint of the first boundary condition. Specifically, the grid structure is formed by dividing the physical model of the blast furnace into a plurality of grid cells. Each grid cell has an unfixed shape and size, but is provided with four grid nodes as vertices. Each grid node can be represented by a radial distance and a vertical distance, and can reflect the fluid radial velocity and fluid vertical velocity of the fluid formed by the furnace burden through reactions inside the blast furnace at the grid node. The blast furnace characteristics include the boundary furnace profile, the blast-furnace center line and the shape of the dead coke bed. FIG. 2 is a schematic diagram of the initial grid structure according to an embodiment of the present application. As shown in FIG. 2, the top stockline is obtained from the stockline coordinates after the current charging operation, and the boundary furnace profile is composed of the furnace shaft, furnace bosh, furnace belly, hearth and raceway depth. The first boundary condition is the boundary formed by the boundary furnace profile, the shape of the dead coke bed, the blast-furnace center line and the stockline coordinates. The physical model of the blast furnace within the boundary formed by the first boundary condition is meshed to obtain the initial grid structure conforming to the blast furnace shape and the furnace burden condition of the current charging operation.

Step S103, based on the stockline coordinates and the descending velocity of the furnace burden, update the initial grid structure to obtain a target grid structure for the current charging operation. Specifically, updating the initial grid structure according to the furnace burden condition of the current charging operation can obtain a target grid structure more in line with the actual internal condition of the blast furnace while taking the dynamic changes of the furnace burden inside the blast furnace into account.

Step S104, cause furnace burden inside the blast furnace to undergo solid-liquid-gas three-phase flow involving mass, momentum and heat transfer, and chemical reactions. When a plurality of furnace burden performance parameters corresponding to each grid node in the target grid structure converge, obtain a distribution situation of each grid cell in the target grid structure and generate a temperature field distribution map, where the distribution situation includes solid temperature, gas temperature, flow rate and pressure in a area where the grid cell is located. Specifically, when the temperature inside the blast furnace reaches a certain threshold, iron ore in the furnace burden begins to soften to form initial melting; as the temperature rises further, part of the furnace burden melts and drips to form the cohesive zone of the blast furnace. Therefore, the temperature factor needs to be focused on when determining the position of the cohesive zone in the blast furnace. Solid-liquid-gas three-phase flow, heat and mass transfer, and chemical reactions mainly occur during the blast furnace ironmaking process, resulting in changes in the mass, momentum and heat of the furnace burden inside the blast furnace. Coupling calculation of the above mass, momentum, heat and chemical reactions is performed at each grid node. When the furnace burden performance parameters of each grid node converge, the distribution situation of each grid cell in the grid structure corresponding to the current charging operation is obtained, and the temperature field distribution map is generated according to the distribution situation of each grid cell to reflect the temperature at each position inside the blast furnace. The furnace burden performance parameters include the edge solid temperature, gas pressure, silicon content, top gas temperature and top gas components. Optionally, other furnace burden performance parameters can be determined according to actual conditions, which are not limited in the embodiment of the present application. The generation of the temperature field distribution map enables accurate determination of the cohesive zone in the blast furnace based on the temperature at each position inside the blast furnace.

Step S105, based on the plurality of furnace burden performance parameters corresponding to each grid node in the target grid structure and the temperature field distribution map, determine the position of the cohesive zone in the blast furnace after the current charging operation. Specifically, the internal condition of the blast furnace can be fully understood through the furnace burden performance parameters corresponding to each grid node and the temperature field distribution map, so that the position of the cohesive zone in the blast furnace can be determined more accurately.

The method for determining the position of the cohesive zone in the blast furnace provided by the embodiment of the present application obtains the stockline coordinates and the descending velocity of the furnace burden for the current charging operation by performing real-time stockline scanning according to the charging start signal and the charging end signal, thereby updating the initial grid structure constructed based on the first boundary condition by virtue of the stockline coordinates and the descending velocity of the furnace burden to obtain the target grid structure. By simulating solid-liquid-gas three-phase flow, heat and mass transfer, and chemical reactions in the grid structure corresponding to the current charging operation, changes of the furnace burden with time and space are taken into account, which enables a deeper understanding of the complex physical and chemical changes inside the blast furnace to further generate the temperature field distribution map. The position of the cohesive zone can be determined more accurately by synthesizing the furnace burden performance parameters and the temperature field distribution map, which provides strong data support for production scheduling and process optimization, improves the accuracy in determining the position of the cohesive zone, and simultaneously reflects the dynamic changes inside the blast furnace.

A method for determining the position of a cohesive zone in a blast furnace is provided in this embodiment, which can be applied to the above-mentioned electronic equipment. The method specifically includes the following steps:
Step S201, perform a stockline scan based on a charging start signal and a charging end signal of a current charging operation to obtain stockline coordinates and a descending velocity of furnace burden for the current charging operation, where the stockline coordinates include coordinates of each point located on a top surface layer of the blast furnace after the end of the current charging operation.

Specifically, the above Step S201 includes:
Step S2011, acquire the charging start signal of the current charging operation when a stock flow valve of the blast furnace is opened. Specifically, charging, i.e., adding coke or ore into the blast furnace, can be performed when the stock flow valve of the blast furnace is opened, and the charging start signal is acquired at this time.

Step S2012, perform a stockline scan based on the charging start signal to obtain initial stockline coordinates for the current charging operation. Specifically, when the charging start signal is acquired, the stockline scan is performed after a preset time delay to make the scanned stockline coordinates more accurate and comprehensive, at which time the charging is stable. The on-line laser stockline scanning device for the blast furnace starts to perform the stockline scan to obtain the initial stockline coordinates. Optionally, the preset time delay and the stockline scanning time can be set arbitrarily, which are not limited in the embodiment of the present application.

Step S2013, acquire the charging end signal of the current charging operation when a sounding rod of the blast furnace is lifted. Specifically, charging is ended, i.e., the addition of coke or ore into the blast furnace is stopped, when the sounding rod of the blast furnace is lifted, and the charging end signal is acquired at this time.

Step S2014, perform a stockline scan based on the charging end signal to obtain the stockline coordinates of the furnace burden for the current charging operation. Specifically, when the charging end signal is acquired, considering that the large amount of dust after the end of charging has an adverse effect on the laser scanning effect of the on-line laser stockline scanning device for the blast furnace, the stockline scan is performed after a preset time delay from the acquisition of the charging end signal to obtain the stockline coordinates of the furnace burden. Optionally, different preset time delays can be set for different types of furnace burden. For example, the preset time delay after the end of coke charging is set to 10 seconds, and the preset time delay after the end of ore charging is set to 20 seconds. Optionally, the preset time delays corresponding to coke and ore above are merely examples, which are not limited in the embodiment of the present application.

Step S2015, determine the descending velocity of the furnace burden for the current charging operation based on the stockline coordinates of the furnace burden for the current charging operation, the initial stockline coordinates, and a time difference between the charging start signal and the charging end signal. Specifically, the height difference between the stockline coordinates of the furnace burden and the initial stockline coordinates is divided by the time difference between the two signals to obtain the descending velocity of the furnace burden. The stockline coordinates are obtained respectively by means of scanning through real-time monitoring of the charging start signal and the charging end signal, and the descending velocity of the furnace burden is determined according to the stockline coordinates obtained by two scans, which provides support for subsequent determination of the position of the cohesive zone.

In some optional embodiments, the central part of the blast furnace is a flame zone, where the laser of the on-line laser stockline scanning device for the blast furnace cannot scan the stockline. Therefore, the coordinates of the central part can be calculated by using the Lagrange interpolation algorithm. The stockline coordinates directly obtained by scanning and the stockline coordinates of the central part obtained by the interpolation algorithm are spliced to form the complete stockline coordinates of the furnace burden.

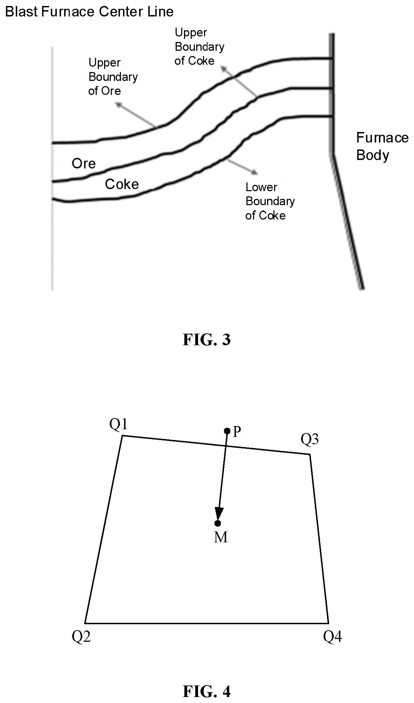

In some optional embodiments, FIG. 3 is a schematic diagram of the distribution of a stockline at the top of a blast furnace according to an embodiment of the present application. As shown in FIG. 3, the stockline coordinates of the furnace burden are acquired in each blast furnace charging operation. For example, ore is charged in the current charging operation, and the stockline coordinates of the furnace burden during the current ore charging operation, i.e., the upper boundary of ore shown in FIG. 3, are obtained through the stockline scan.

Step S202. Determine a first boundary condition based on the stockline coordinates and blast furnace characteristics, and construct an initial grid structure based on the first boundary condition, where the initial grid structure is obtained by meshing a physical model of the blast furnace under the constraint of the first boundary condition. For details, refer to Step S102 in the embodiment shown in FIG. 1, which will not be repeated herein.

Step S203, update the initial grid structure based on the stockline coordinates and the descending velocity of the furnace burden to obtain a target grid structure for the current charging operation.

Specifically, the above Step S203 includes:
Step S2031, determine grid parameters of each grid node in the initial grid structure, where the grid parameters include a radial distance, a vertical distance, a fluid radial velocity and a fluid vertical velocity.

In some optional embodiments, the above Step S2031 includes:
Step a1, perform coordinate transformation on the stockline coordinates to obtain grid parameters of each point in the stockline coordinates. Specifically, coordinate transformation is performed on the stockline coordinates of the furnace burden for the current charging operation through the Laplace equation, and the grid parameters of each grid node in the initial grid structure corresponding to the stockline coordinates are calculated by the following formula (1) according to the finite difference method in a Cartesian coordinate system. Optionally, the above process is all the prior art, which will not be repeated herein.$\frac{{\partial }^{2} ψ}{\partial {r}^{2}} - \frac{1}{r} \frac{\partial ψ}{\partial r} + \frac{{\partial }^{2} ψ}{\partial {z}^{2}} = 0$
where *ψ* represents the stream function; *r* represents the radial distance; *z* represents the vertical distance.

Step a2, determine the grid parameters of each grid node in the initial grid structure based on the grid parameters of each point in the stockline coordinates. Specifically, since the stockline coordinates are obtained by scanning the top stockline of the blast furnace, they correspond to the top grid nodes of the initial grid structure in the initial grid structure. After the grid parameters of each grid node at the top are determined, the grid parameters of other grid nodes in the initial grid structure except the top grid nodes can be calculated by using the prior art, for example, adopting the convection trajectory equation and starting from the known top grid nodes in accordance with the physical characteristics and motion law of the furnace burden. The specific process will not be repeated herein.

Step S2032, for any grid node in the initial grid structure that corresponds to the stockline coordinates, cause the grid node to descend and determine grid parameters of a descended grid node obtained after the descent.

In some optional embodiments, the above Step S2032 includes:
Step b1, determine a descent time of the grid node. Specifically, it is assumed that any grid node is caused to descend, and the descent time is preset. Optionally, in the subsequent repeated descent process, the embodiment of the present application is described by taking the same descent time adopted for each descent as an example.

Step b2, determine a radial distance of the descended grid node based on the radial distance, the fluid radial velocity of the grid node and the descent time. Specifically, the radial distance of the descended grid node of the grid node can be determined by the following formula (2).$r \left({i}_{a}, {j}_{a}\right) = r \left({i}_{b}, {j}_{b}\right) + νr \left({i}_{b}, {j}_{b}\right) * t$
where (*iₐ*, *jₐ*) represents the index of the descended grid node located in the *iₐ* row and the *jₐ* column; (*i_{b}*, *j_{b}*) represents the index of the grid node located in the *i_{b}* row and the *j_{b}* column; r(*iₐ*, *jₐ*) represents the radial distance of the descended grid node located in the *iₐ* row and the *jₐ* column; r(*i_{b}*, *j_{b}*) represents the radial distance of the grid node located in the *i_{b}* row and the *j_{b}* column; *vr*(*i_{b}*, *j_{b}*) represents the fluid radial velocity of the grid node located in the *i_{b}* row and the *j_{b}* column; *t* represents the descent time.

Step b3, determine a vertical distance of the descended grid node based on the vertical distance, the fluid vertical velocity of the grid node and the descent time. Specifically, the vertical distance of the descended grid node of the grid node can be determined by the following formula (3).$z \left({i}_{a}, {j}_{a}\right) = z \left({i}_{b}, {j}_{b}\right) + νz \left({i}_{b}, {j}_{b}\right) * t$
where (*iₐ*, *jₐ*) represents the index of the descended grid node located in the *iₐ* row and the *jₐ* column; (*i_{b}*, *j_{b}*) represents the index of the grid node located in the *i_{b}* row and the *j_{b}* column; z(*iₐ*, *jₐ*) represents the vertical distance of the descended grid node located in the *iₐ* row and the *jₐ* column; *z*(*i_{b}*, *j_{b}*) represents the vertical distance of the grid node located in the *i_{b}* row and the *j_{b}* column; vz(*i_{b}*, *j_{b}*) represents the fluid vertical velocity of the grid node located in the *i_{b}* row and the *j_{b}* column; *t* represents the descent time.

Step b4, determine a grid cell to which the descended grid node belongs in the initial grid structure based on the radial distance and the vertical distance of the descended grid node. Specifically, FIG. 4 is a schematic diagram of a grid cell according to an embodiment of the present application. As shown in FIG. 4, it is assumed that point P is a grid node and point M is the descended grid node of point P, and the grid cell where point M is located is determined from the initial grid structure, i.e., the grid cell formed by Q1-Q2-Q3-Q4.

Step b5, determine four distances between the descended grid node and four vertex grid nodes of the grid cell based on the grid parameters of the grid node, the radial distance and the vertical distance of the descended grid node, and the grid parameters of the four vertex grid nodes of the grid cell. Specifically, the grid cell where point M is located is Q1-Q2-Q3-Q4, and the distances between point M and the four vertices of the grid cell, i.e., the distances from point M to point Q1, point Q2, point Q3 and point Q4 respectively, are determined by the following formulas (4) to (7).${s}_{1} = \sqrt{{\left[r\left({i}_{1}, {j}_{1}\right)-nr\left({i}_{M}, {j}_{M}\right)\right]}^{2} + {\left[z\left({i}_{1}, {j}_{1}\right)-nz\left({i}_{M}, {j}_{M}\right)\right]}^{2}}$${s}_{2} = \sqrt{{\left[r\left({i}_{2}, {j}_{2}\right)-nr\left({i}_{M}, {j}_{M}\right)\right]}^{2} + {\left[z\left({i}_{2}, {j}_{2}\right)-nz\left({i}_{M}, {j}_{M}\right)\right]}^{2}}$${s}_{3} = \sqrt{{\left[r\left({i}_{3}, {j}_{3}\right)-nr\left({i}_{M}, {j}_{M}\right)\right]}^{2} + {\left[z\left({i}_{3}, {j}_{3}\right)-nz\left({i}_{M}, {j}_{M}\right)\right]}^{2}}$${s}_{4} = \sqrt{{\left[r\left({i}_{4}, {j}_{4}\right)-nr\left({i}_{M}, {j}_{M}\right)\right]}^{2} + {\left[z\left({i}_{4}, {j}_{4}\right)-nz\left({i}_{M}, {j}_{M}\right)\right]}^{2}}$
where *s*₁ represents the distance between point M and point Q1; *s*₂ represents the distance between point M and point Q2; *s*₃ represents the distance between point M and point Q3; *s*₄ represents the distance between point M and point Q4; *r*(*i*₁, *j*₁) represents the radial distance of point Q1 located in the *i*₁ row and the *j*₁ column; *nr*(*i_{M}*, *j_{M}*) represents the abscissa of point M in the grid cell Q1-Q2-Q3-Q4; *z*(*i*₁, *j*₁) represents the vertical distance of point Q1 located in the *i*₁ row and the *j*₁ column; *nz*(*i_{M}*, *j_{M}*) represents the ordinate of point M in the grid cell Q1-Q2-Q3-Q4; *r*(*i*₂, *j*₂) represents the radial distance of point Q2 located in the *i*₂ row and the *j*₂ column; *z*(*i*₂, *j*₂) represents the vertical distance of point Q2 located in the *i*₂ row and the *j*₂ column; *r*(*i*₃, *j*₃) represents the radial distance of point Q3 located in the *i*₃ row and the *j*₃ column; *z*(*i*₃, *j*₃) represents the vertical distance of point Q3 located in the *i*₃ row and the *j*₃ column; *r*(*i*₄, *j₄*) represents the radial distance of point Q4 located in the *i*₄ row and the *j*₄ column; *z*(*i*₄, *j*₄) represents the vertical distance of point Q4 located in the *i*₄ row and the *j*₄ column.

Step b6, Determine the fluid radial velocity and the fluid vertical velocity of the descended grid node based on the grid parameters of the four vertex grid nodes of the grid cell and the four distances. Specifically, the fluid radial velocity and the fluid vertical velocity of the descended grid node can be determined by constructing an interpolation function through the following formulas (8) and (9). The sinking behavior of the furnace burden inside the blast furnace can be simulated by calculating the grid parameters of the descended grid node obtained after the descent of the grid node, which provides intuitive visual and quantitative data support for understanding the dynamic changes inside the blast furnace.$νr \left({i}_{M}, {j}_{M}\right) = \frac{νr \left({i}_{i}, {j}_{i}\right) \frac{1}{{s}_{1}} + νr \left({i}_{2}, {j}_{2}\right) \frac{1}{{s}_{2}} + νr \left({i}_{3}, {j}_{3}\right) \frac{1}{{s}_{3}} + νr \left({i}_{4}, {j}_{4}\right) \frac{1}{{s}_{4}}}{\frac{1}{{s}_{1}} + \frac{1}{{s}_{2}} + \frac{1}{{s}_{3}} + \frac{1}{{s}_{4}}}$$νz \left({i}_{M}, {j}_{M}\right) = \frac{νz \left({i}_{i}, {j}_{i}\right) \frac{1}{{s}_{1}} + νz \left({i}_{2}, {j}_{2}\right) \frac{1}{{s}_{2}} + νz \left({i}_{3}, {j}_{3}\right) \frac{1}{{s}_{3}} + νz \left({i}_{4}, {j}_{4}\right) \frac{1}{{s}_{4}}}{\frac{1}{{s}_{1}} + \frac{1}{{s}_{2}} + \frac{1}{{s}_{3}} + \frac{1}{{s}_{4}}}$
where *vr*(*i_{M}*, *j_{M}*) represents the fluid radial velocity of point M located in the *i_{M}* row and the *j_{M}* column; *vz*(*i_{M}*, *j_{M}*) represents the fluid vertical velocity of point M located in the *i_{M}* row and the *j_{M}* column; *vr*(*i*₁, *j*₁) represents the fluid radial velocity of point Q1 located in the *i*₁ row and the *j*₁ column; *vz*(*i*₁, *j*₁) represents the fluid vertical velocity of point Q1 located in the *i*₁ row and the *j*₁ column; *s*₁ represents the distance between point M and point Q1; *vr*(*i*₂, *j*₂) represents the fluid radial velocity of point Q2 located in the *i*₂ row and the *j*₂ column; *vz*(*i*₂, *j*₂) represents the fluid vertical velocity of point Q2 located in the *i*₂ row and the *j*₂ column; *s*₂ represents the distance between point M and point Q2; *vr*(*i*₃, *j*₃) represents the fluid radial velocity of point Q3 located in the *i*₃ row and the *j*₃ column; *vz*(*i*₃, *j*₃) represents the fluid vertical velocity of point Q3 located in the *i*₃ row and the *j*₃ column; *s*₃ represents the distance between point M and point Q3; *vr*(*i*₄, *j*₄) represents the fluid radial velocity of point Q4 located in the *i*₄ row and the *j*₄ column; *vz*(*i*₄, *j*₄) represents the fluid vertical velocity of point Q4 located in the *i*₄ row and the *j*₄ column; *s*₄ represents the distance between point M and point Q4.

Step S2033, take the descended grid node as a new grid node and repeat the process of descending the grid node and acquiring the grid parameters of the descended grid node until the grid node or the descended grid node exceeds a physical domain, thereby obtaining grid parameters of at least one descended grid node corresponding to the grid node. Specifically, each grid node at the top in the initial grid structure is caused to descend in the initial grid structure, a descended grid node and its grid parameters are determined after the descent time, then the descended grid node is taken as a new grid node to continue descending, and the next descended grid node and its grid parameters are determined. The above process is repeated until the grid node or the descended grid node exceeds the physical domain, so as to obtain the grid parameters of all descended grid nodes corresponding to each grid node at the top of the initial grid structure. The physical domain can refer to the grid area shown in FIG. 2.

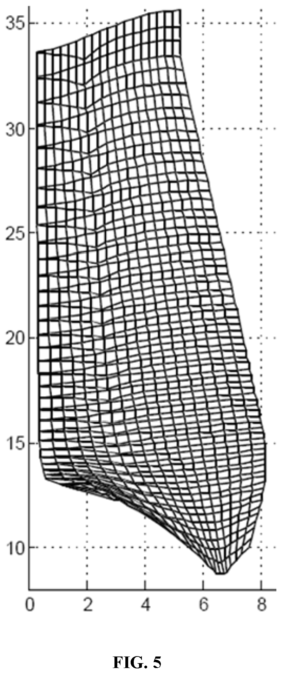

Step S2034, update the grid parameters of each grid node in the initial grid structure based on the grid parameters of the at least one descended grid node obtained during the descending process for each grid node in the initial grid structure that corresponds to the stockline coordinates, to obtain the target grid structure. Specifically, for each grid node in the initial grid structure corresponding to the stockline coordinates, i.e., each grid node at the top of the initial grid structure, the grid parameters of the grid node in the initial grid structure at the same position as the descended grid node are updated correspondingly based on the grid parameters of all descended grid nodes obtained by simulating the descent of the furnace burden for each grid node. This simulates the descending process of the furnace burden with time and space, can reflect the dynamic change situation inside the blast furnace, and obtains the target grid structure conforming to the actual situation of the current charging operation, which provides support for subsequent determination of the position of the cohesive zone. FIG. 5 is a schematic diagram of the target grid structure according to an embodiment of the present application. The target grid structure shown in FIG. 5 is more in line with the actual situation of the current charging operation compared with the initial grid structure shown in FIG. 2.

Step S204, cause furnace burden inside the blast furnace to undergo solid-liquid-gas three-phase flow involving mass, momentum and heat transfer, and chemical reactions. When a plurality of furnace burden performance parameters corresponding to each grid node in the target grid structure converge, obtain a distribution situation of each grid cell in the target grid structure and generate a temperature field distribution map, where the distribution situation includes solid temperature, gas temperature, flow rate and pressure in an area where the grid cell is located.

Specifically, the above Step S204 includes:
Step S2041, determine a second boundary condition. Specifically, the furnace body thermocouple, furnace shaft static pressure, molten iron composition, top gas temperature and top gas components are taken as the second boundary condition. The furnace body thermocouple can be obtained through the Fourier heat conduction equation, i.e., the following formula (10), which represents the temperature of the solid furnace burden at the furnace wall; the molten iron composition refers to the actual silicon content in the molten iron.$\frac{dq}{du} = - kA \frac{dU}{dx}$
where *q* represents the heat quantity; *u* represents the time; *k* represents the thermal conductivity; *A* represents the furnace body area; *U* represents the furnace body temperature; *x* represents the furnace body length.

Step S2042, cause the furnace burden inside the blast furnace to undergo the solid-liquid-gas three-phase flow, heat and mass transfer, and chemical reactions for the first time to obtain the plurality of furnace burden performance parameters corresponding to each grid node in the target grid structure and the distribution situation of each grid cell after the first solid-liquid-gas three-phase flow, heat and mass transfer, and chemical reactions. Specifically, the furnace burden inside the blast furnace undergoes solid-liquid-gas three-phase flow, heat and mass transfer, and chemical reactions during the blast furnace ironmaking process. The solid-liquid-gas three-phase flow, heat and mass transfer involves the mass, momentum and heat transfer of the furnace burden, and the chemical reactions involve the reduction reactions of iron, carbon and silicon. The furnace burden performance parameters correspond to the above second boundary condition, including the edge solid temperature, gas pressure, silicon content, as well as the top gas temperature and top gas components. The solid-liquid-gas three-phase flow, heat and mass transfer, and chemical reactions are caused to occur for the first time inside the blast furnace to obtain the furnace burden performance parameters of each grid node and the distribution situation of each grid cell after the first reaction. More specifically, the reduction reaction of iron refers to the chemical reaction equation shown in the following formula (11), the reduction reaction of carbon refers to the chemical reaction equation shown in the following formula (12), and the reduction reaction of silicon refers to the chemical reaction equation shown in the following formula (13). 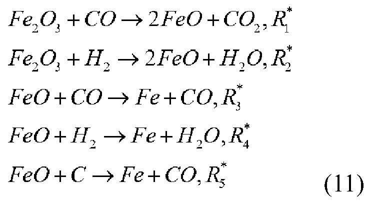 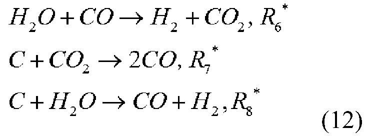 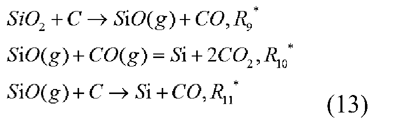 where *Fe*₂*O*₃ is iron (III) oxide; *CO* is carbon monoxide; *CO*(*g*) is gaseous carbon monoxide; *FeO* is iron(II) oxide; *CO*₂ is carbon dioxide; *H*₂ is hydrogen; *H*₂*O* is water; *Fe* is iron; *C* is carbon; *S*i is silicon; *SiO*₂ is silicon dioxide; *S*i*O*(*g*) is gaseous silicon monoxide; each *R*^{*} represents the rate of the corresponding chemical reaction.

Step S2043, repeat the process of causing the furnace burden inside the blast furnace to undergo the solid-liquid-gas three-phase flow, heat and mass transfer, and chemical reactions until the plurality of furnace burden performance parameters corresponding to each grid node reach convergence under the constraint of the second boundary condition, thereby obtaining the distribution situation of each grid cell in the target grid structure and generating the temperature field distribution map. Specifically, after the first occurrence of solid-liquid-gas three-phase flow, heat and mass transfer, and chemical reactions inside the blast furnace, it is judged whether the furnace burden performance parameters of each grid node after the first reaction reach convergence under the constraint of the second boundary condition, i.e., it is judged whether the errors between the edge solid temperature and the set value of the furnace body thermocouple, between the gas pressure and the set value of the furnace shaft static pressure, between the silicon content and the set value of the molten iron composition, and between the top gas temperature and components in the furnace burden performance parameters and the set values of the top gas temperature and components in the second boundary condition are all within a preset error range. If the error of each item of the furnace burden performance parameters of each grid node from the corresponding item in the second boundary condition is within the preset error range, convergence is deemed to be achieved. If any grid node does not reach convergence after the first reaction, the solid-liquid-gas three-phase flow, heat and mass transfer, and chemical reactions are performed for the second time, and the judgment on whether the furnace burden performance parameters after the second reaction converge is continued. The process is repeated until each grid node converges after any time of solid-liquid-gas three-phase flow, heat and mass transfer, and chemical reactions, and then the temperature field distribution map is generated based on the distribution situation of each grid cell obtained after the reaction. The temperature field distribution map can intuitively display the temperature, flow rate and pressure at each position inside the blast furnace. The distribution situation of each grid cell is obtained by setting the second boundary condition and repeatedly performing the simulation of three-phase flow mass transfer and chemical reactions until the furnace burden performance parameters converge, so as to generate the temperature field distribution map. The temperature field distribution map intuitively shows the spatial distribution of temperature inside the blast furnace, which provides support for subsequent determination of the position of the cohesive zone.

Step S205, determine the position of the cohesive zone in the blast furnace after the current charging operation based on the plurality of furnace burden performance parameters corresponding to each grid node in the target grid structure and the temperature field distribution map.

Specifically, the above Step S205 includes:
Step S2051, perform high-temperature droplet performance detection based on the plurality of furnace burden performance parameters corresponding to each grid node in the target grid structure to obtain an initial softening temperature and an initial dripping temperature of the furnace burden. Specifically, the high-temperature droplet performance detection is a material testing method that focuses on the morphology, melting characteristics, flow behavior and mechanical properties of molten droplets at high temperatures, and thus can be used to determine the initial softening temperature and initial dripping temperature of the furnace burden. The plurality of furnace burden performance parameters corresponding to each grid node when convergence is achieved in the above Step S2043 are taken as the environmental parameters in the high-temperature droplet performance detection, i.e., the detection is performed in the environment corresponding to the furnace burden performance parameters, to obtain the initial softening temperature and initial dripping temperature of the furnace burden.

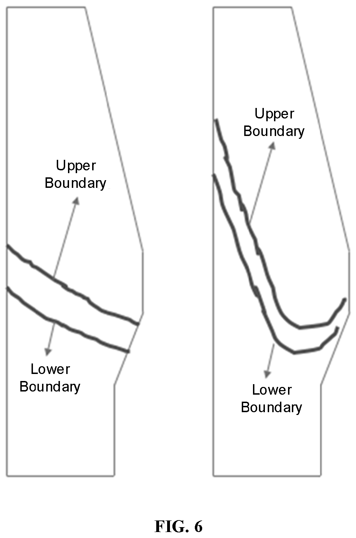

Step S2052, based on temperatures of all grid cells in the target grid structure in the temperature field distribution map, take all grid cells whose temperatures fall between the initial softening temperature and the initial dripping temperature as the position of the cohesive zone in the blast furnace after the current charging operation. Specifically, since the temperature field distribution map displays the temperature at each position inside the blast furnace, the grid cells in the target grid structure whose temperatures are between the two temperatures are determined as the position of the cohesive zone, achieving accurate determination of the position of the cohesive zone. FIG. 6 is a schematic diagram of a cohesive zone in a blast furnace according to an embodiment of the present application. As shown in FIG. 6, the left side is a center-suppressed cohesive zone of the blast furnace, and the right side is an ideal cohesive zone of the blast furnace. The shape of the cohesive zone of the blast furnace is not fixed, and the one shown in FIG. 6 is merely an example.

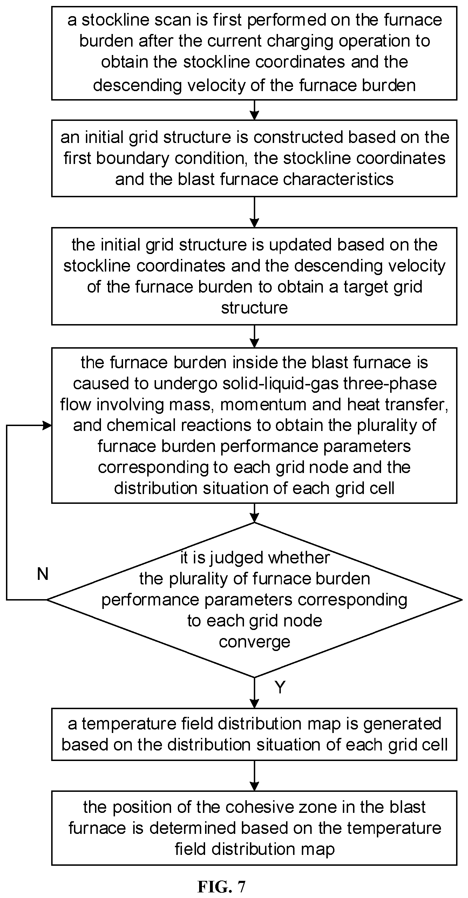

In some optional embodiments, FIG. 7 is a flow chart of another method for determining the position of a cohesive zone in a blast furnace according to an embodiment of the present application. As shown in FIG. 7, a stockline scan is first performed on the furnace burden after the current charging operation to obtain the stockline coordinates and the descending velocity of the furnace burden. Then, an initial grid structure is constructed based on the first boundary condition, the stockline coordinates and the blast furnace characteristics. Then, the initial grid structure is updated based on the stockline coordinates and the descending velocity of the furnace burden to obtain a target grid structure. Then, the furnace burden inside the blast furnace is caused to undergo solid-liquid-gas three-phase flow involving mass, momentum and heat transfer, and chemical reactions to obtain the plurality of furnace burden performance parameters corresponding to each grid node and the distribution situation of each grid cell. Then, it is judged whether the plurality of furnace burden performance parameters corresponding to each grid node converge based on the second boundary condition. If convergence is achieved, a temperature field distribution map is generated based on the distribution situation of each grid cell. If convergence is not achieved, the process returns to the step of causing the furnace burden inside the blast furnace to undergo solid-liquid-gas three-phase flow involving mass, momentum and heat transfer, and chemical reactions. Finally, the position of the cohesive zone in the blast furnace is determined based on the temperature field distribution map.

The method for determining the position of the cohesive zone in the blast furnace provided by the embodiment of the present application obtains the stockline coordinates and the descending velocity of the furnace burden for the current charging operation by performing real-time stockline scanning according to the charging start signal and the charging end signal, thereby updating the initial grid structure constructed based on the first boundary condition by virtue of the stockline coordinates and the descending velocity of the furnace burden to obtain the target grid structure. By simulating solid-liquid-gas three-phase flow, heat and mass transfer, and chemical reactions in the grid structure corresponding to the current charging operation, changes of the furnace burden with time and space are taken into account, which enables a deeper understanding of the complex physical and chemical changes inside the blast furnace to further generate the temperature field distribution map. The position of the cohesive zone can be determined more accurately by synthesizing the furnace burden performance parameters and the temperature field distribution map, which provides strong data support for production scheduling and process optimization, improves the accuracy in determining the position of the cohesive zone, and simultaneously reflects the dynamic changes inside the blast furnace.

An apparatus for determining the position of a cohesive zone in a blast furnace is also provided in this embodiment, which is used to implement the above embodiments and preferred implementation manners. The contents that have been described will not be repeated herein. As used hereinafter, the term "module" may refer to a combination of software and/or hardware that can implement a predetermined function. Although the apparatus described in the following embodiments is preferably implemented by software, implementation by hardware or a combination of software and hardware is also possible and contemplated.

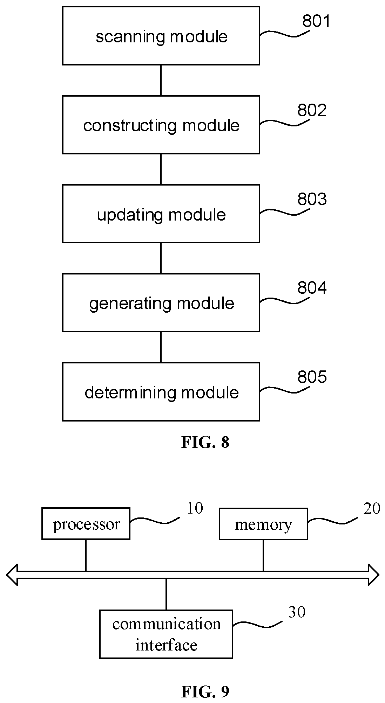

An apparatus for determining the position of a cohesive zone in a blast furnace is provided in this embodiment. As shown in FIG. 8, the apparatus includes:
a scanning module 801, configured to perform a stockline scan based on a charging start signal and a charging end signal of a current charging operation to obtain stockline coordinates and a descending velocity of furnace burden for the current charging operation, where the stockline coordinates include coordinates of each point located on a top surface layer of the blast furnace after the end of the current charging operation;
a constructing module 802, configured to determine a first boundary condition based on the stockline coordinates and blast furnace characteristics, and to construct an initial grid structure based on the first boundary condition, where the initial grid structure is obtained by meshing a physical model of the blast furnace under the constraint of the first boundary condition;
an updating module 803, configured to update the initial grid structure based on the stockline coordinates and the descending velocity of the furnace burden to obtain a target grid structure for the current charging operation;
a generating module 804, configured to cause furnace burden inside the blast furnace to undergo solid-liquid-gas three-phase flow involving mass, momentum and heat transfer, and chemical reactions, and when a plurality of furnace burden performance parameters corresponding to each grid node in the target grid structure converge, to obtain a distribution situation of each grid cell in the target grid structure and generate a temperature field distribution map, where the distribution situation includes solid temperature, gas temperature, flow rate and pressure in an area where the grid cell is located; and
a determining module 805, configured to determine the position of the cohesive zone in the blast furnace after the current charging operation based on the plurality of furnace burden performance parameters corresponding to each grid node in the target grid structure and the temperature field distribution map.

In some optional embodiments, the scanning module 801 includes:
a first acquiring unit, configured to acquire the charging start signal of the current charging operation when a stock flow valve of the blast furnace is opened;
a first scanning unit, configured to perform a stockline scan based on the charging start signal to obtain initial stockline coordinates for the current charging operation;
a second acquiring unit, configured to acquire the charging end signal of the current charging operation when a sounding rod of the blast furnace is lifted;
a second scanning unit, configured to perform a stockline scan based on the charging end signal to obtain the stockline coordinates of the furnace burden for the current charging operation; and
a first determining unit, configured to determine the descending velocity of the furnace burden for the current charging operation based on the stockline coordinates of the furnace burden for the current charging operation, the initial stockline coordinates, and a time difference between the charging start signal and the charging end signal.

In some optional embodiments, the updating module 803 includes:
a second determining unit, configured to determine grid parameters of each grid node in the initial grid structure, where the grid parameters include a radial distance, a vertical distance, a fluid radial velocity and a fluid vertical velocity;
a descending unit, configured to for any grid node in the initial grid structure that corresponds to the stockline coordinates, cause the grid node to descend and determine grid parameters of a descended grid node obtained after the descent;
a third determining unit, configured to take the descended grid node as a new grid node and repeat the process of descending the grid node and acquiring the grid parameters of the descended grid node until the grid node or the descended grid node exceeds a physical domain, thereby obtaining grid parameters of at least one descended grid node corresponding to the grid node; and
an updating unit, configured to update the grid parameters of each grid node in the initial grid structure based on the grid parameters of the at least one descended grid node obtained during the descending process for each grid node in the initial grid structure that corresponds to the stockline coordinates, to obtain the target grid structure.

In some optional embodiments, the second determining unit includes:
a transforming subunit, configured to perform coordinate transformation on the stockline coordinates to obtain grid parameters of each point in the stockline coordinates; and
a first determining subunit, configured to determine the grid parameters of each grid node in the initial grid structure based on the grid parameters of each point in the stockline coordinates.

In some optional embodiments, the descending unit includes:
a second determining subunit, configured to determine a descent time of the grid node;
a third determining subunit, configured to determine a radial distance of the descended grid node based on the radial distance of the grid node, the fluid radial velocity, and the descent time;
a fourth determining subunit, configured to determine a vertical distance of the descended grid node based on the vertical distance of the grid node, the fluid vertical velocity, and the descent time;
a fifth determining subunit, configured to determine a grid cell to which the descended grid node belongs in the initial grid structure based on the radial distance and the vertical distance of the descended grid node;
a sixth determining subunit, configured to determine four distances between the descended grid node and four vertex grid nodes of the grid cell based on the grid parameters of the grid node, the radial distance and the vertical distance of the descended grid node, and the grid parameters of the four vertex grid nodes of the grid cell; and
a seventh determining subunit, configured to determine the fluid radial velocity and the fluid vertical velocity of the descended grid node based on the grid parameters of the four vertex grid nodes of the grid cell and the four distances.

In some optional embodiments, the generating module 804 includes:
a fourth determining unit, configured to determine a second boundary condition;
a reacting unit, configured to cause the furnace burden inside the blast furnace to undergo the solid-liquid-gas three-phase flow, heat and mass transfer, and chemical reactions for the first time to obtain the plurality of furnace burden performance parameters corresponding to each grid node in the target grid structure and the distribution situation of each grid cell after the first solid-liquid-gas three-phase flow, heat and mass transfer, and chemical reactions; and
a generating unit, configured to repeat the process of causing the furnace burden inside the blast furnace to undergo the solid-liquid-gas three-phase flow, heat and mass transfer, and chemical reactions until the plurality of furnace burden performance parameters corresponding to each grid node reach convergence under the constraint of the second boundary condition, thereby obtaining the distribution situation of each grid cell in the target grid structure and generating the temperature field distribution map.

In some optional embodiments, the determining module 805 includes:
a detecting unit, configured to perform high-temperature droplet performance detection based on the plurality of furnace burden performance parameters corresponding to each grid node in the target grid structure to obtain an initial softening temperature and an initial dripping temperature of the furnace burden; and
a fifth determining unit configured to, based on temperatures of all grid cells in the target grid structure in the temperature field distribution map, take all grid cells whose temperatures fall between the initial softening temperature and the initial dripping temperature as the position of the cohesive zone in the blast furnace after the current charging operation.

For further functional descriptions of the above modules and units, refer to the corresponding embodiments above, which will not be repeated herein.

The apparatus for determining the position of a cohesive zone in a blast furnace in this embodiment is presented in the form of functional units, where the unit refers to an ASIC (Application Specific Integrated Circuit) circuit, a processor and a memory that execute one or more software or fixed programs, and/or other devices that can provide the above functions.

An embodiment of the present application also provides a computer device provided with the above apparatus for determining the position of a cohesive zone in a blast furnace shown in FIG. 8.

Please refer to FIG. 9, which is a schematic diagram of a hardware structure of a computer device according to an optional embodiment of the present application. As shown in FIG. 9, the computer device includes: one or more processors 10, a memory 20, and interfaces for connecting various components, including a high-speed interface and a low-speed interface. The various components are communicatively connected to each other by using different buses, and can be mounted on a common motherboard or mounted in other ways as required. The processor can process instructions executed in the computer device, including instructions stored in or on the memory to display graphic information of a GUI on an external input/output device (such as a display device coupled to the interface). In some optional embodiments, multiple processors and/or multiple buses can be used together with multiple memories and multiple memories if required. Similarly, multiple computer devices can be connected, and each device provides part of the necessary operations (for example, as a server array, a group of blade servers, or a multiprocessor system). One processor 10 is taken as an example in FIG. 9.

The processor 10 may be a central processing unit (CPU), a network processor (NP) or a combination thereof. The processor 10 may further include a hardware chip. The above hardware chip may be an application-specific integrated circuit (ASIC), a programmable logic device (PLD) or a combination thereof. The above programmable logic device may be a complex programmable logic device (CPLD), a field-programmable gate array (FPGA), a generic array logic (GAL) or any combination thereof.

The memory 20 stores instructions executable by at least one processor 10 to enable the at least one processor 10 to implement the method shown in the above embodiments.

The memory 20 may include a program storage area and a data storage area, where the program storage area may store an operating system and an application program required for at least one function; the data storage area may store data created according to the use of the computer device, and the like. In addition, the memory 20 may include a high-speed random access memory, and may also include a non-volatile memory, such as at least one magnetic disk storage device, a flash memory device, or other non-volatile solid-state storage devices. In some optional embodiments, the memory 20 may optionally include a memory remotely disposed relative to the processor 10, and these remote memories may be connected to the computer device through a network. Examples of the above network include, but are not limited to, the Internet, an intranet, a local area network, a mobile communication network, and combinations thereof.

The memory 20 may include a volatile memory, such as a random access memory (RAM); the memory may also include a non-volatile memory, such as a flash memory, a hard disk or a solid-state disk; the memory 20 may also include a combination of the above types of memories.

The computer device further includes a communication interface 30, configured for the computer device to communicate with other devices or a communication network.

An embodiment of the present application also provides a computer-readable storage medium. The method according to the embodiment of the present application described above can be implemented in hardware, firmware, or implemented as software code that can be recorded on a storage medium, or implemented as computer code originally stored on a remote storage medium or a non-transitory machine-readable storage medium downloaded through a network and to be stored on a local storage medium, so that the method described herein can be processed by software stored on a storage medium using a general-purpose computer, a dedicated processor, or programmable or dedicated hardware. The storage medium may be a magnetic disk, an optical disc, a read-only memory (ROM), a random access memory (RAM), a flash memory, a hard disk or a solid-state disk, etc.; further, the storage medium may also include a combination of the above types of memories. It can be understood that a computer, a processor, a microprocessor controller or programmable hardware includes a storage component that can store or receive software or computer code, and when the software or computer code is accessed and executed by the computer, processor or hardware, the method shown in the above embodiments is implemented.

A part of the present application can be applied as a computer program product, such as computer program instructions. When executed by a computer, the computer program instructions can call or provide the method and/or technical solution according to the present application through the operation of the computer. Those skilled in the art can understand that the existence forms of computer program instructions in a computer-readable medium include, but are not limited to, source files, executable files, installation package files, etc. Correspondingly, the execution manners of the computer program instructions by the computer include, but are not limited to the computer directly executes the instructions, or the computer compiles the instructions and then executes the corresponding compiled program, or the computer reads and executes the instructions, or the computer reads and installs the instructions and then executes the corresponding installed program. Herein, the computer-readable medium may be any available computer-readable storage medium or communication medium accessible to a computer.

Although the embodiments of the present application have been described in conjunction with the accompanying drawings, those skilled in the art can make various modifications and variations without departing from the spirit and scope of the present application, and such modifications and variations all fall within the protection scope defined by the present application.

## Claims

1. A method for determining a position of a cohesive zone in a blast furnace comprising:
based on a charging start signal and a charging end signal of a current charging operation by using an on-line laser stockline scanning device for the blast furnace, performing a stockline scan to obtain stockline coordinates and a descending velocity of furnace burden for the current charging operation, wherein the stockline coordinates comprise coordinates of each point located on a top surface layer of the blast furnace after the end of the current charging operation, the stockline coordinates of the central part of the blast furnace stockline are calculated by a Lagrange interpolation algorithm based on the stockline coordinates scanned by the on-line laser stockline scanning device for the blast furnace, and the stockline coordinates comprise the stockline coordinates scanned by the on-line laser stockline scanning device for the blast furnace and the stockline coordinates of the central part of the blast furnace stockline;
based on the stockline coordinates and blast furnace characteristics, determining a first boundary condition, and constructing an initial grid structure based on the first boundary condition, wherein the initial grid structure is obtained by meshing a physical model of the blast furnace under the constraint of the first boundary condition, and the blast furnace characteristics comprise a boundary furnace profile, a blast-furnace center line and a shape of a dead coke bed;
based on the stockline coordinates and the descending velocity of the furnace burden, updating the initial grid structure to obtain a target grid structure for the current charging operation;
causing furnace burden inside the blast furnace to undergo solid-liquid-gas three-phase flow involving mass, momentum and heat transfer, and chemical reactions, and when a plurality of furnace burden performance parameters corresponding to each grid node in the target grid structure converge, obtaining a distribution situation of each grid cell in the target grid structure and generating a temperature field distribution map, wherein the distribution situation comprises solid temperature, gas temperature, flow rate, and pressure in an area where the grid cell is located; and
based on the plurality of furnace burden performance parameters corresponding to each grid node in the target grid structure and the temperature field distribution map, determining the position of the cohesive zone in the blast furnace after the current charging operation;
wherein the performing a stockline scan based on the charging start signal and the charging end signal of the current charging operation to obtain the stockline coordinates and the descending velocity of the furnace burden for the current charging operation comprises:
when a stock flow valve of the blast furnace is opened, acquiring the charging start signal of the current charging operation;
performing a stockline scan based on the charging start signal to obtain initial stockline coordinates for the current charging operation;
when a sounding rod of the blast furnace is lifted, acquiring the charging end signal of the current charging operation;
performing a stockline scan based on the charging end signal to obtain the stockline coordinates of the furnace burden for the current charging operation; and
determining the descending velocity of the furnace burden for the current charging operation based on the stockline coordinates of the furnace burden for the current charging operation, the initial stockline coordinates, and a time difference between the charging start signal and the charging end signal, the descending velocity of the furnace burden is a quotient obtained by dividing the height difference between the stockline coordinates of the furnace burden and the initial stockline coordinates by the time difference;
the step of updating the initial grid structure based on the stockline coordinates and the descending velocity of the furnace burden to obtain the target grid structure for the current charging operation comprises:
determining grid parameters of each grid node in the initial grid structure, wherein the grid parameters comprise a radial distance, a vertical distance, a fluid radial velocity, and a fluid vertical velocity;
for any grid node in the initial grid structure that corresponds to the stockline coordinates, causing the grid node to descend and determining grid parameters of a descended grid node obtained after the descent;
taking the descended grid node as a new grid node and repeating the process of descending the grid node and acquiring the grid parameters of the descended grid node until the grid node or the descended grid node exceeds a physical domain, thereby obtaining grid parameters of at least one descended grid node corresponding to the grid node; and
updating the grid parameters of each grid node in the initial grid structure based on the grid parameters of the at least one descended grid node obtained during the descending process for each grid node in the initial grid structure that corresponds to the stockline coordinates, to obtain the target grid structure;
the step of causing any grid node corresponding to the stockline coordinates in the initial grid structure to descend and determining the grid parameters of the descended grid node obtained after the descent comprises:
determining a descent time of the grid node;
determining a radial distance of the descended grid node based on the radial distance of the grid node, the fluid radial velocity, and the descent time;
determining a vertical distance of the descended grid node based on the vertical distance of the grid node, the fluid vertical velocity, and the descent time;
determining a grid cell to which the descended grid node belongs in the initial grid structure based on the radial distance and the vertical distance of the descended grid node;
determining four distances between the descended grid node and four vertex grid nodes of the grid cell based on the grid parameters of the grid node, the radial distance and the vertical distance of the descended grid node, and the grid parameters of the four vertex grid nodes of the grid cell; and
determining the fluid radial velocity and the fluid vertical velocity of the descended grid node based on the grid parameters of the four vertex grid nodes of the grid cell and the four distances.

2. The method according to claim 1, wherein the determining the grid parameters of each grid node in the initial grid structure comprises:
performing coordinate transformation on the stockline coordinates to obtain grid parameters of each point in the stockline coordinates; and
determining the grid parameters of each grid node in the initial grid structure based on the grid parameters of each point in the stockline coordinates.

3. The method according to claim 1, wherein the causing furnace burden inside the blast furnace to undergo solid-liquid-gas three-phase flow involving mass, momentum and heat transfer, and chemical reactions, and when the plurality of furnace burden performance parameters corresponding to each grid node in the target grid structure converge, obtaining the distribution situation of each grid cell in the target grid structure and generating the temperature field distribution map comprises:
determining a second boundary condition;
causing the furnace burden inside the blast furnace to undergo the solid-liquid-gas three-phase flow, heat and mass transfer, and chemical reactions for the first time to obtain the plurality of furnace burden performance parameters corresponding to each grid node in the target grid structure and the distribution situation of each grid cell after the first solid-liquid-gas three-phase flow, heat and mass transfer, and chemical reactions; and
repeating the process of causing the furnace burden inside the blast furnace to undergo the solid-liquid-gas three-phase flow, heat and mass transfer, and chemical reactions until the plurality of furnace burden performance parameters corresponding to each grid node reach convergence under the constraint of the second boundary condition, thereby obtaining the distribution situation of each grid cell in the target grid structure and generating the temperature field distribution map.

4. The method according to claim 1, wherein the determining the position of the cohesive zone in the blast furnace after the current charging operation based on the plurality of furnace burden performance parameters corresponding to each grid node in the target grid structure and the temperature field distribution map comprises:
performing high-temperature droplet performance detection based on the plurality of furnace burden performance parameters corresponding to each grid node in the target grid structure to obtain an initial softening temperature and an initial dripping temperature of the furnace burden; and
based on the temperatures of all grid cells in the target grid structure in the temperature field distribution map, taking all grid cells whose temperatures fall between the initial softening temperature and the initial dripping temperature as the position of the cohesive zone in the blast furnace after the current charging operation.

5. An apparatus for determining a position of a cohesive zone in a blast furnace, the apparatus comprising:
a scanning module configured to perform a stockline scan based on a charging start signal and a charging end signal of a current charging operation by using an on-line laser stockline scanning device for the blast furnace to obtain stockline coordinates and a descending velocity of furnace burden for the current charging operation, wherein the stockline coordinates comprise coordinates of each point located on a top surface layer of the blast furnace after the end of the current charging operation, the stockline coordinates of the central part of the blast furnace stockline are calculated by a Lagrange interpolation algorithm based on the stockline coordinates scanned by the on-line laser stockline scanning device for the blast furnace, and the stockline coordinates comprise the stockline coordinates scanned by the on-line laser stockline scanning device for the blast furnace and the stockline coordinates of the central part of the blast furnace stockline;
a constructing module configured to determine a first boundary condition based on the stockline coordinates and blast furnace characteristics, and to construct an initial grid structure based on the first boundary condition, wherein the initial grid structure is obtained by meshing a physical model of the blast furnace under the constraint of the first boundary condition, and the blast furnace characteristics comprise a boundary furnace profile, a blast-furnace center line and a shape of a dead coke bed;
an updating module configured to update the initial grid structure based on the stockline coordinates and the descending velocity of the furnace burden to obtain a target grid structure for the current charging operation;
a generating module configured to cause furnace burden inside the blast furnace to undergo solid-liquid-gas three-phase flow involving mass, momentum and heat transfer, and chemical reactions, and when a plurality of furnace burden performance parameters corresponding to each grid node in the target grid structure converge, to obtain a distribution situation of each grid cell in the target grid structure and generate a temperature field distribution map, wherein the distribution situation comprises solid temperature, gas temperature, flow rate, and pressure in an area where the grid cell is located; and
a determining module configured to determine the position of the cohesive zone in the blast furnace after the current charging operation based on the plurality of furnace burden performance parameters corresponding to each grid node in the target grid structure and the temperature field distribution map;
the scanning module is specifically configured to:
acquire the charging start signal of the current charging operation when a stock flow valve of the blast furnace is opened;
perform a stockline scan based on the charging start signal to obtain initial stockline coordinates for the current charging operation;
acquire the charging end signal of the current charging operation when a sounding rod of the blast furnace is lifted;
perform a stockline scan based on the charging end signal to obtain the stockline coordinates of the furnace burden for the current charging operation; and
determine the descending velocity of the furnace burden for the current charging operation based on the stockline coordinates of the furnace burden for the current charging operation, the initial stockline coordinates, and a time difference between the charging start signal and the charging end signal, the descending velocity of the furnace burden is a quotient obtained by dividing the height difference between the stockline coordinates of the furnace burden and the initial stockline coordinates by the time difference;
the updating module is specifically configured to:
determine grid parameters of each grid node in the initial grid structure, wherein the grid parameters comprise a radial distance, a vertical distance, a fluid radial velocity, and a fluid vertical velocity;
for any grid node in the initial grid structure that corresponds to the stockline coordinates, cause the grid node to descend and determine grid parameters of a descended grid node obtained after the descent;
take the descended grid node as a new grid node and repeat the process of descending the grid node and acquiring the grid parameters of the descended grid node until the grid node or the descended grid node exceeds a physical domain, thereby obtaining grid parameters of at least one descended grid node corresponding to the grid node; and
update the grid parameters of each grid node in the initial grid structure based on the grid parameters of the at least one descended grid node obtained during the descending process for each grid node in the initial grid structure that corresponds to the stockline coordinates, to obtain the target grid structure;
for any grid node in the initial grid structure that corresponds to the stockline coordinates, cause the grid node to descend and determine grid parameters of a descended grid node obtained after the descent comprising:
determining a descent time of the grid node;
determining a radial distance of the descended grid node based on the radial distance of the grid node, the fluid radial velocity, and the descent time;
determining a vertical distance of the descended grid node based on the vertical distance of the grid node, the fluid vertical velocity, and the descent time;
determining a grid cell to which the descended grid node belongs in the initial grid structure based on the radial distance and the vertical distance of the descended grid node;
determining four distances between the descended grid node and four vertex grid nodes of the grid cell based on the grid parameters of the grid node, the radial distance and the vertical distance of the descended grid node, and the grid parameters of the four vertex grid nodes of the grid cell; and
determining the fluid radial velocity and the fluid vertical velocity of the descended grid node based on the grid parameters of the four vertex grid nodes of the grid cell and the four distances.

6. A computer device, comprising:
a memory and a processor that are communicatively connected to each other, wherein the memory stores computer instructions, and the processor executes the computer instructions to perform the method for determining the position of a cohesive zone in a blast furnace according to any one of claims 1 to 4.

7. A computer-readable storage medium, wherein the computer-readable storage medium stores computer instructions that, when executed by a computer, cause the computer to perform the method for determining the position of a cohesive zone in a blast furnace according to any one of claims 1 to 4.
